# EUROPEAN PATENT APPLICATION

(11) **EP 0 628 636 A1**
(43) Date of publication of application: **14.12.1994**
(21) Application number: 93201617.3
(22) Date of filing: 07.06.1993
(51) Int. Cl.: C12N 15/82, C12N 15/54, A01H 5/00

(54) **Transgenic plants exhibiting modified carbohydrate metabolism**

(71) Applicant: STICHTING AGROTECHNOLOGISCH ONDERZOEK ( ATO-DLO), NL-6708 PM Wageningen (NL)
(72) Inventor: Claassen, Pieternel Arnolda Maria, NL-6704 PM Wageningen (NL); Gouveia, Maria Manuela Camare, NL-6702 BX Wageningen (NL); Mooibroek, Andreas, NL-6871 TT Renkum (NL); Huizing, Hindrik Jan, NL-6703 DW Wageningen (NL); Budde, Miriam Anna Wilhelmina, NL-6658 GK Beneden-Leeuwen (NL)
(74) Representative: de Bruijn, Leendert C.

(57) **Abstract**

The increase in concentration of sucrose and hexoses or other low molecular weight metabolites during the exposure of plant tissue to low temperatures is a widespread phenomenon. Sweetened plant material, however, becomes unsuitable for processing because of the Maillard reaction between reducing sugars and amino acids. The subject invention is directed at solving this problem. The subject invention concerns a method for reducing plant material's ability for producing sugars which can participate in Maillard reactions or precursors of said sugars, wherein at least one nucleotide sequence is introduced into the plant material, said nucleotide sequence expressing a product capable of reducing the amount of UDPG available for conversion to sucrose and wherein the reduction in sugar production occurs upon application of an external stimulus. The subject invention is also directed at a recombinant plant material exhibiting reduced sucrose formation characterised by the presence of at least one nucleotide sequence encoding at least a part of anti-sense pyrophosphate fructosephosphate phosphotransferase (PFP) mRNA, said part being sufficient to cause inhibition of production of pyrophosphate fructosephosphate phosphotransferase (PFP) after application of an external stimulus.

## Description

### FIELD OF THE INVENTION

The present invention relates to a method for the reduction of sugar accumulation in plant material. In particular the invention is directed at the development of transgenic plants which show a modified sugar production due to their genetically altered carbohydrate and/or pyrophosphate metabolism.

### BACKGROUND OF THE INVENTION

The increase in concentration of sucrose and hexoses or other low molecular weight metabolites during the exposure of plant tissue to low temperatures is a widespread phenomenon. The capacity to produce these metabolites following cold-stress assumedly contributes to frost hardiness of these plants (ap Rees et al., 1981).

In potato tubers, e.g. sucrose and reducing sugars accumulate readily during storage at temperatures below 8°C, the exact temperature depending on variety and growth conditions. Sweetened potatoes, however, become unsuitable for processing because of the Maillard reaction between reducing sugars and amino acids, and the subject invention is directed at solving this problem. In particular our research was aimed at the investigation of fundamental aspects of sweetening and at the development of potato varieties which produce tubers with an increased low temperature tolerance. This way storability of agricultural produce becomes independent of the use of chemical sprout suppressants or disease suppressing agents.

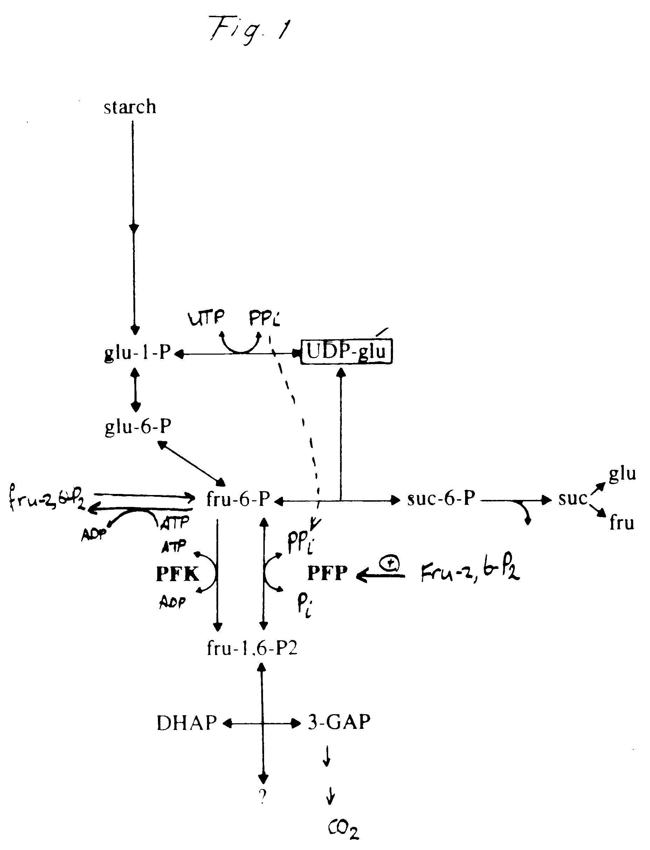

In many previous studies, a block in glycolysis due to cold lability of two enzymes catalyzing the conversion of Fru-6-P to Fru-1,6-P₂ has been suggested as the cause of a metabolic switch towards sucrose synthesis instead of glycolysis and respiration at reduced temperatures (Trevanion and Kruger, 1991) (figure 1). These two enzymes are phosphofructokinase (PFK: EC 2.7.1.11.) and pyrophosphate fructose phosphate phosphotransferase (PFP:EC 2.7.1.90). It has generally been assumed that PFK controls the glycolytic flux as it catalyzes a non-equilibrium reac-PFK controls the glycolytic flux as it catalyzes a non-equilibrium reaction. The function of the second enzyme, pyrophoshate fructosephoshate phosphotransferase (PFP: EC 2.7.1.90) has been the subject of a hitherto unsolved debate as PFP may participate either in glycolysis or gluconeogenesis (Kruger 1990).

The occurrence of a block in glycolysis causing a metabolic switch towards sucrose synthesis, however, until now has appeared unlikely. Firstly because respiration of tubers proceeds unimpeded when stored in the cold (Isherwood 1973). Secondly because we have shown that the specific activity of PFP *in vitro* does not change at low temperatures and is seemingly high enough to enable maintenance conversion of starch to CO₂ through the glycolytic and respiratory pathways (Claassen et al 1991).

After applying fluoride to spinach leaves Quick et al. (1989) observed that sucrose synthesis is heavily dependent upon the intracellular concentration of PPi, as a more than twofold increase in PPi concentration was concurrent with a decrease in sucrose content. The basis of the effect of fluoride on the endogenous PPi concentration was not clarified, but the authors dismissed the inhibition of PFP leading to increased PPi levels due to it's apparent lack of fluoride sensitivity. These authors indicated that fluoride might interfere with the hydrolysis of PPi, possibly by interference with the PPi-dependent proton pump in the tonoplast membrane. Interference with the PPi metabolism could therefore lead to serious undesirable distortions in the membrane proton pump.

Indication of the involvement of PPi in carbohydrate metabolism has been provided by Jelitto et al (1992). The basis of the effect of low PPi concentrations on sucrose concentration they observed was ascribed to inhibition of sucrose mobilisation via sucrose synthase and UDPG pyrophosphorylase. No change was observed in the ratio of hexose-P: triose-P indicating that low PPi concentrations did not apparently additionally disrupt carbohydrate metabolism at alternative metabolic steps. Inhibition of the conversion step of Fru-6-P to Fru-1,6-P₂ by PFP due to reduced PPi levels was consequently discarded by Jellitto et al. (1992). It was suggested that a lower PEP activity was either compensated for by increased PFK activity converting Fru-6-P to Fru-1,6-P₂ or that PFP was stimulated more by the increased Fru-2,6-P₂ content.

It is generally known that the cellular concentration of PPi is dependent on a combination of a number of PPi generating and PPi utilizing reactions. The most important PPi generating reactions are part of core biosynthetic pathways mainly occurring in specialized subcellular compartments. Examples of PPi generating reactions are adenylation of glucose-1-P in polysaccharide biosynthesis, the activation of fatty acids, the citydylation of P-choline and P-ethanolamine in lipid biosynthesis, the coupling of isopentenyl-PP moieties in isoprenoid biosynthesis, the aminoacylation of t-RNA in polypeptide synthesis and the formation of phosphodiester bonds in polynucleotide synthesis.

In the cytoplasm, the main reactions involved in PPi turnover are the hydrolysis by a H⁺-translocating pyrophosphatase in the tonoplast membrane, the uridinylation of Glu-1-P by UDPG pyrophosphorylase and the phosphorylation of Fru-6-P by PFP.

Pyrophosphatase catalyzes the hydrolysis of PPi to two molecules of Pi. The release of energy from this reaction is presumed to pull other metabolic reactions to completion. The location of this tonoplast-pyrophosphatase provides an extra dimension with respect to cellular energy as this membrane-bound enzyme can generate a proton motive force. This way, the energy liberated during PPi hydrolysis is not lost as heat but is conserved (Rea et al., 1992). By contrast, the free energy relationship for the hydrolysis of PPi shows that the pyrophosphatase can also be involved in PPi synthesis (Rea and Sanders, 1987). The stoichiometry of the number of protons translocated for each PPi hydrolysed and the pH difference between the cytosol and the vacuole will determine the direction of the reaction. When PPi is synthesized (at the cost of ATP) the physiological meaning of pyrophosphatase could be the stabilization of cytosolic PPi levels.

Both the uridinylation and the phosphorylation reactions are reversible and show mass-action ratios which are close to the equilibrium constant (Weiner et al. 1987). This means that their contribution to the turnover of PPi is governed by the relative concentrations of substrates and products of the respective reaction.

Jellitto et al (1992) discovered that sucrose synthesis can be increased by increasing the pyrophosphatase activity through transformation with E.coli pyrophosphatase and thus illustrated a link between sucrose formation and PPi. However, as stated, cellular energy may be derived for a major part from hydrolysis of PPi (Rea et al., 1992). Interfering in the PPi metabolism therefore was expected to have disastrous consequences, seriously disrupting cellular metabolism or at least the fitness of the plant.

Other important cytosolic reactions have also been postulated as utilizing or supplying PPi, for example the phosphorylation steps converting Fru-6-P to Fru-1,6-P₂ in the glycolytic pathway and vice versa in the gluconeogenetic direction. It was considered plausible that suppression of PFP activity for the latter could result in prevention of production of PPi in this reaction and subsequently prevent mobilisation of sucrose via sucrose synthase and UDPG phosphorylase resulting indirectly in accumulation of sucrose.

On the other hand suppression of PFP activity in the glycolytic pathway could lead to accumulation of PPi derived from UDPG synthesis. This could result in the following potential drawbacks. First glycolysis would become completely dependent on PFK activity and it is questionable whether this would be sufficient to provide for respiration or to prevent either accumulation of Fru-6-P and subsequent sucrose formation or accumulation of Fru-2,6-P₂. Secondly it would be entirely feasible that the endogenous pyrophosphatase activity could neutralize the increase in PPi at the Fru-6-P to Fru-1,6-P₂ conversion step and therefore have no effect on the conversion step of Glu-1-P to UDPG.

Suppression of PFP activity could therefore result in an aggravation of sucrose accumulation due to the increased Fru-6-P concentration and the annihilation of the control by PPi. In the worst scenario a plant in which PFP activity is reduced and in which the cold labile PFK is subsequently also inactivated by cold could die after exposure to low temperatures due to hampered glycolysis.

### DESCRIPTION OF THE FIGURES

Figure 1 shows an overview of a part of the sugar metabolism.

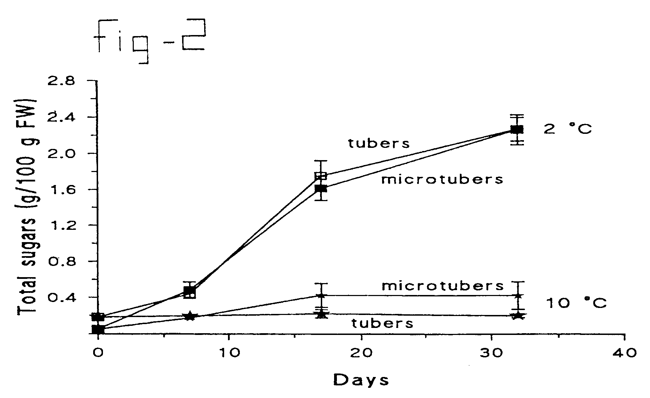

Figure 2 shows the accumulation of sugars in tubers and microtubers of Solanum tuberosum cv. Saturna during storage at 2 or 10°C.

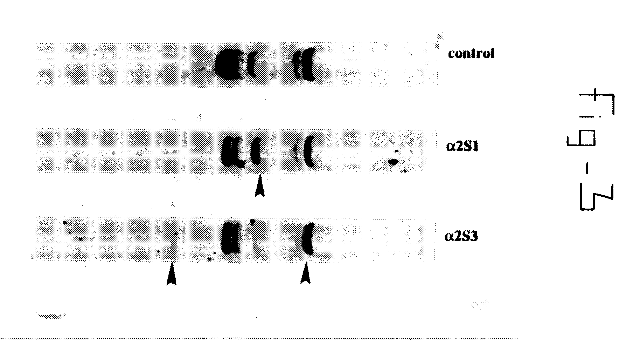

Figure 3 is the Southern blot analysis of Solanum tuberosum cv. Saturna transformed with antisense PFP_{β}-constructs. Total genomic DNA was digested using XbaI restriction enzyme. Control DNA is obtained from non-transformed plantlets.

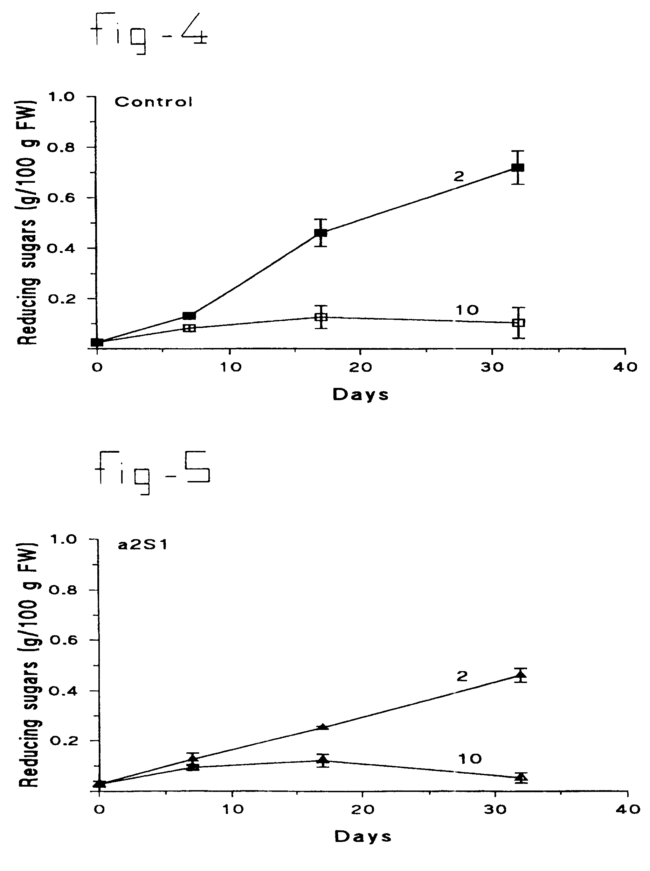

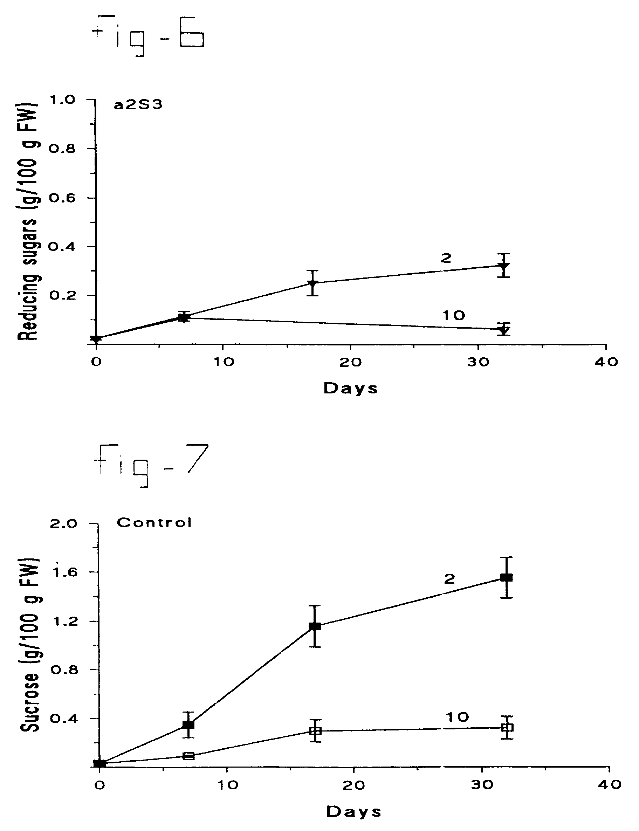

Figures 4, 5 and 6 illustrate the accumulation of reducing sugars in microtubers of control and transformed plantlets of Solanum tuberosum cv. Saturna during storage at 2 or 10°C. Transformed plantlets contained 3' 600 base pairs of PFP_{β} cloned in the antisense direction.

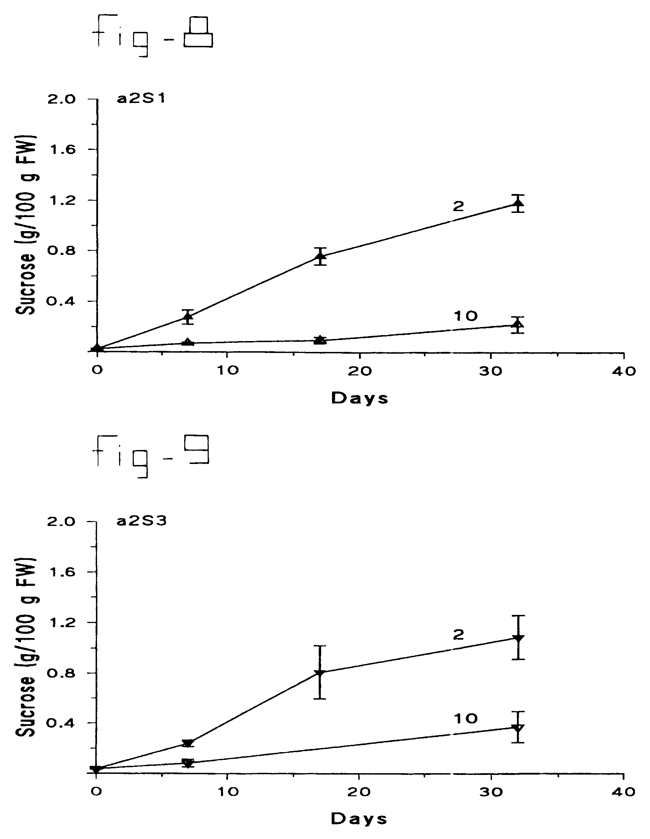

Figures 7, 8 and 9 illustrate accumulation of sucrose in microtubers of control and transformed plantlets of Solanum tuberosum cv. Saturna during storage at 2 or 10°C. Transformed plantlets contained 3' 600 base pairs of PFP_{β}, cloned in the antisense direction.

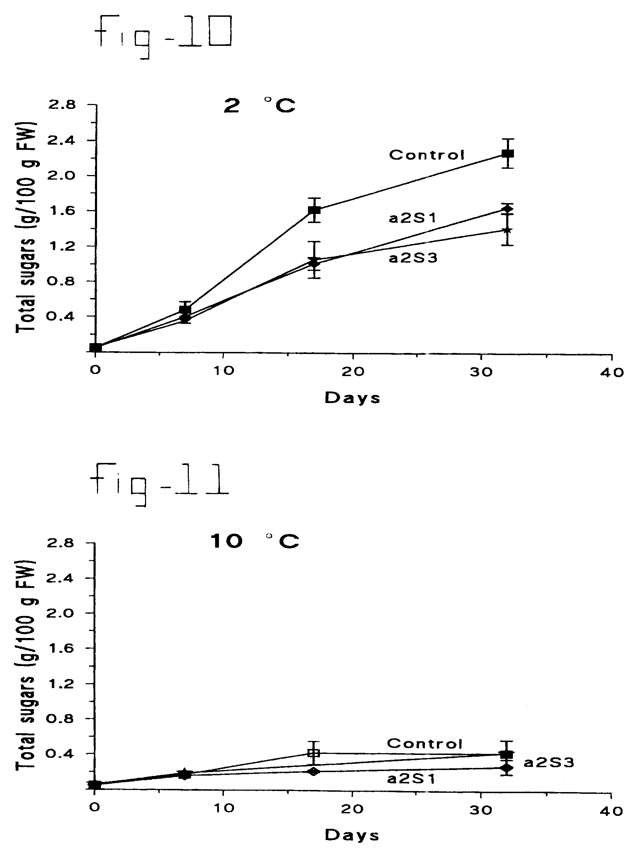

Figures 10 and 11 illustrate the total sugar concentration in micro-tubers of control and antisense PFB_{β} transformed plantlets of Solanum tuberosum cv. Saturna during storage at 2 or 10°C.

### DESCRIPTION OF THE INVENTION

In view of the above it is therefore surprising that good results have been achieved with the method according to the subject invention.

The subject invention is directed at a method for reducing plant material's ability for producing sugars which can participate in Maillard reactions or precursors of said sugars wherein at least one nucleotide sequence is introduced into the plant material, said nucleotide sequence expressing a product capable of reducing the amount of UDPG available for conversion to sucrose and wherein the reduction in sugar production occurs upon application of an external stimulus.

In particular it was surprising to find that interfering with PPi metabolism leads to a decrease in sucrose formation without concomitant disastrous or deleterious consequences for the plant material. The reduction of the plant material's ability for producing sucrose can be obtained by inhibiting the conversion of fructose-6-phosphate to fructose-1,6-P₂ via pyrophosphate fructose phosphate phosphotransferase (PFP). This apparently leads to sufficient increase in the amount of available cytosolic pyrophosphate to suppress UDPG formation and subsequently to reduce sucrose formation.

A preferred embodiment of the method according to the invention comprises introduction of at least one nucleotide sequence encoding at least a part of anti-sense pyrophosphate fructose phosphate phosphotransferase (PFP) sufficient to ensure inhibition of production of pyrophosphate fructose phosphate phosphotransferase. The sequence encoding PFP is described in Carlisle et al (1990).

Any equivalent sequence capable of sufficient hybridisation to prevent expression of PFP can be incorporated in the anti-sense direction in a method according to the invention. The use of anti-sense nucleotide sequences to inhibit activity of particular gene products is a technique well known to a person skilled in the art and there will be no problem ascertaining what anti-sense sequences are potentially useful for the method according to the invention. An example of this embodiment of the method according to the invention is given later in this description.

As already stated the method according to the invention requires an external stimulus to be applied to the plant material to activate and maintain the reduced sucrose formation. In the case where non-transformed plants are susceptible to cold sweetening the external stimulus to be applied to the corresponding transgenic plant according to the invention can simply be a temperature below the threshold value of the temperature leading to cold sweetening in the non-transformed plant. A suitable temperature as external stimulus for the method according to the invention in particular when the plant material is potato material is a temperature below the threshold temperature which suppresses sprout growth of the particular non-transformed potato variety. This temperature is generally below 8°C and for a number of varieties this is below 6°C. In particular the invention is directed at production of transgenic potatoes in the manner described and application of temperatures below the aforementioned threshold temperature as the external stimulus. It is also a specific embodiment of the invention that the nucleotide sequences that are incorporated can be subjected to control via the external stimulus. This is e.g. possible by incorporating an inducible promoter for the nucleotide sequence expressing a product capable of reducing the amount of UDPG available for conversion to sucrose. The external stimulus that is subsequently to be applied in the method according to the invention thus is the inducer for said promoter.

Another preferred embodiment of the method according to the invention comprises incorporating a tuber specific promoter controlling the nucleotide suquence expressing a product capable of reducing the amount of UDPG available for conversion to sucrose. In this manner it is possible to leave the sucrose formation of the plant itself unaltered in comparison to the corresponding non-transformed plant, whilst obtaining altered sucrose formation behavior for the tubers of the transformed plant upon application of the external stimulus in comparison to the tubers of the non-transformed plant.

The invention is in particular directed at altering plant material of commercial value, in which plant material or parts thereof products having UDPG as precursor accumulate, said alteration being brought about in the manners indicated above.

The subject invention is also directed at recombinant plant material comprising one or more of the nucleotide sequences and optionally the promoters as described above for the various embodiments of the method according to the invention. The invention is also directed at the offspring of such recombinant plant material and parts of said plant material. Transgenic tubers for example produced by the transgenic plants according to the invention, can find application as new produce with a modified solid content, a modified taste and an altered storage potential.

In particular a specific embodiment of the invention is directed at recombinant plant material exhibiting reduced sucrose formation characterised in the presence of at least one nucleotide sequence encoding at least a part of anti-sense pyrophosphate fructosephosphate phosphotransferase (PFP) mRNA, said part being sufficient to cause inhibition of production of pyrophosphate fructosephosphate phosphotransferase (PFP).

Plant material to be used for the method according to the invention and to be used for preparing transgenic plant material having the desired characteristics is potentially economically interesting agricultural produce which is stored at low temperatures in order to maintain quality such as potato plants and in particular potato tubers. The most preferred potato species are potato species used for preparing foodstuffs destined for cooking, in particular those used for crisp and chip making or production of other types of snacks based on dried or fresh potato granules.

Examples of preferred varieties are Bintje, Hertha, Saturna, Marijke, Agria, van Gogh, Morene, Erntestolz, Lady Rosetta, Record, Pentland Dell, Asterix, Disco, Eba, Amazone, Allure and Brodick.

The present invention in particular provides transgenic plant material, e.g. plants and plant organs, which possess an altered response with respect to their carbohydrate metabolism after a decrease in temperature. The production of such plants can take place via the introduction into the plant of at least one nucleotide sequence as described above encoding a product which interferes with sugar metabolism. Preferably this genetic modification is without obvious effect on plant vitality at ambient temperatures and creates new agricultural produce with new processing properties and/or different flavours.

The sink organs of the transgenic plants according to the invention show a reduced accumulation of sucrose and reducing sugars compared with the non-transformed plants. This modified phenotype can be created by the introduction of DNA constructs which modify the PPi metabolism. The expression of these DNA sequences can be placed under the control of the CaMV35S promotor which promoter can also be replaced by other regulatory sequences capable of directing the expression of the gene products at a specific tissue or become active at certain developmental stages or environmental conditions.

Specifically the invention is directed at the use of a nucleotide sequence producing at least a part of anti-sense pyrophosphate fructosephosphate phosphotransferase (PFP) mRNA for obtaining recombinant plant material exhibiting reduced sucrose formation, said part being sufficient to ensure inhibition of production of pyrophosphate fructosephosphate phosphotransferase (PFP).

### DETAILED DESCRIPTION OF MATERIALS AND METHODS

### Plant material

Shoot cultures of potato (Solanum tuberosum cv. Saturna) were grown in vitro on MS medium (Murashige and Skoog, 1962) at 24°C, at 16h day-length, and micropropagated by subculturing stem/leaf segments containing apical or nodal meristematic tissue every 4-5 weeks.

### Transformation vectors

Antisense (Carlisle et al., 1990) PFPβ was cloned into pBI121 after removing the coding region of the GUS (β-glucuronidase) reporter gene. Three vectors containing the entire PFPβ (1.5kb), a 3'- (0.6kb) or a 5'-(0.7kb) were ligeted between the CaMV35S-promoter and the nopaline synthase polyadenylation site.

Introduction of the plasmids into A.tumefaciens LBA4404 was performed by electroporation using a Gene Pulser apparatus (BioRad) and the following parameters: shunt resistance 200Ω, voltage 2.5kV, capacity 25µF in 0.2cm cuvettes. Transformants were selected for kanamycin resistance. The integrity of vectors was verified by restiction analysis of minipreps from A.tumefaciens cultures and by Southern blot analysis.

### Potato transformation

For transformation stem explants were excised from 4-5 week old shoot cultures and co-cultivated with A.tumefaciens following the modified protocol of Horsch et al (1985) as described by Visser (1991). In short, explants were precultured for 1 day on petridishes containing basal solidified MS medium supplemented with sucrose (2%), Zeatin (1 mg.L⁻¹) and NAA (0.01 mg.L⁻¹), overlayed with 2 mL of MS medium supplemented with 2,4-D (2 mg.L⁻¹), Kinetin (0.5 mg.L⁻¹), caseinhydrolysate (2 g.L⁻¹), pH=6.5 and covered with a sterile filter paper.

Explants were dipped in a diluted (1:2 in LB medium) overnight A.tumefaciens culture for 5-10 min, blotted dry and transferred back to the same media. After 2 days and every 2 weeks the explants were transferred to selection medium: MS medium containing Zeatin-riboside (1 mg.L⁻ ¹), kanamycin (100 mg.L⁻¹) and cefotaxim (200 mg.L⁻¹). Regenerated shoots were transferred to selection medium without Zeatin-riboside and transformation was assessed by continued growth and rooting in the presence of kanamycin (100 mg.L⁻¹).

### In vitro tuberisation

To induce microtubers single-node segments from axenically grown plants were cultured on basal MS medium supplemented with sucrose (6%), BAP (1 mg.L⁻¹), and solidified with Gelrite (0.2%). Tuberisation took place in the dark at 24°C and microtubers were collected after 4 weeks. The average fresh weight of the microtubers used in this particular experiment ranged from 35 to 73 mg per microtuber.

### Plant DNA isolation

Total plant DNA was extracted from freeze-dried and ground stem/leaf tissue (20 mg) as described (Raeder and Broda, 1985). The DNA was digested with restriction enzymes, electrophoresed in agarose (0.8%, 10µg of DNA per lane), transferred to positively charged nylon membranes (Boehringer) and hybridised with a PFPβ digoxiginin probe, which was prepared by PCR according to Lion and Haas (1990). Membranes were shaken for 4 h at 37°C in solution containing 50%, formamide, 5 * SSC, 2% blocking reagent (Boehringer), 7% SDS, 50 mM NaPi buffer and 0.1% sarcosyl. The hybridization was performed overnight at 37°C in the same solution containing heat-denatured Dig-11-dUT-labelled PFPβ probe. The filters were washed and the DNA hybrids were detected using the AMPPD autoluminescense system (Boehringer).

### Enzyme assay

Crude enzyme extracts were prepared from triplicate samples of microtubers using approximately 200 mg of fresh weight in a modified 50 mM Tris-HCl buffer (pH=8.0) containing 2 mM EDTA and 1 mM PMSF according to Claassen et al. (1991). Extractions were carried out on ice for 10 min and after centrifugation the homogenates were desalted on a column (0.5 x 10 cm) of Bio-Gel P-6DG (BioRad) equilibrated in extraction buffer minus PMSF.

PFP activity was measured spectrophotometrically at 30°C as described previously (Claassen et al., 1991). Protein concentration was determined according to Bradford (1976) using BSA fraction V as a standard.

Specific activity of PFP in shoots and microtubers of control and transformed plantlets of Solanum tuberosum cv. Saturna is shown in the following table.

The activity of PFP in the microtubers was determined at the beginning of the storage experiment.

**Table**

| Specific activity of PFP (nmol. min⁻¹.mg⁻¹ protein) | | |
|---|---|---|
| | plantlets | microtubers |
| control | 16.64 | 17.46 ± 6.11 |
| transformant (a2S1) | 4.55 | 2.49 ± 1.64 |
| transformant (a2S3) | N.D. | 7.82 ± 5.78 |

### Sugar determination

Freeze-dried powder (20-30 mg) from triplicate samples of microtubers (corresponding to approximately 200 mg fresh weight), was extracted with 1 mL of 80% (v/v) ethanol for 2h at 70°C (Claassen et al., 1992). The sucrose, glucose and fructose contents were then measured enzymatically according to a test kit protocol (Boehringer) or using a microplate reader system (Bio Rad) according to Viola and Davies (1992).

### REFERENCES

Carlisle SM, Blakely SD, Hemmingsen SM, Trevanion SJ, Hiyoshi T, Kruger NJ, Dennis DT (1990) Pyrophosphate-dependent phosphofructokinase. J Biol Chem 265:18366-18371
Claassen PAM, Budde MAW, de Ruyter HJ, van Calker MH, van Es A (1991) Potential role of pyrophosphate:fructose 6-phosphate phosphotransferase in carbohydrate metabolism of cold stored tubers of Solanum tuberosum cv Bintje. Plant Physiol 95:1243-1249
Claassen PAM, van Calker MH, Marinus J (1992) Accumulation of sugars in microtubers of potato node cuttings (cv Kennebec) during cold storage. Pot Research 35:191-194
Horsch R.B., Fry J.E., Hoffmann N.L., Eichholtz D., Rogers S.G. and Fraley R.T. (1985). A simple and general method for transferring genes into plants. Science 227:1229-1231
Isherwood FA (1973) Starch-sugar interconversion in Solanum tuberosum. Phytochemistry 12:2579-2591
Jelitto T, Sonnewald U, Willmitzer L, Hajirezeai, Stitt M (1992) Inorganic pyrophosphate content and metabolites in potato and tobacco plants expressing E. coli pyrophosphatase in their cytosol. Planta 188:238-244
Kruger NJ (1990) Carbohydrate synthesis and degradation. In: (DT Dennis and DH Turpin, Eds) Plant Physiology, Biochemistry and Molecular Biology, Longman Scientific & Technical, Harlow, England, pp 59-77
Lion T. and Haas O.A. (1989). Nonradioactive labelling of probe with digoxigenin by polymerase chain reaction. An. Biochem. 188:335-337.
Murashige T and Skoog F (1962). A revised medium for rapid growth aqnd bioassays with tobacco tissue culture. Physiologia Plantarum 15:473-497
Quick P, Neuhaus E, Feil R and Stitt M (1989) Fluoride leads to an increase of inorganic pyrophoaphate and an inhibition of photosynthetic sucrose synthesis in spinach leaves. Biochim Biophys Acta 973:263-271
Rea PA, Yongcheol K, Sarafian V, Poole RJ, Davies JM and Sanders D (1992). Vacuolar H⁺-translocating pyrophosphatases: a new category of ion translocase. TIBS 17:348-353
Rea PA and Sanders D (1987) Tonoplast energization: Two H⁺ pumps, one membrane. Physiol Plantarum 71:131-141
Reader U. and Broda P. (1985). Rapid preparation of the DNA from filamentous fungi. tt. Applied Microbiol. 1:17-20.
ap Rees T, Dixon WL, Pollock CJ, Franks F (1981) Low temperature sweetening of higher plants. In J Friend, MJC Rhodes, eds, Recent Advances in the Biochemistry of Fruits and Vegetables. Academic Press, New York, pp 41-61
Trevanion SJ, Kruger NJ (1991) Effect of temperature on the kinetic properties of pyrophosphate:fructose 6-phosphate phosphotransferase from potato tuber. J Plant Physiol 137:753-759
Viola R. and Davies H.V. (1992). A microplate reader assay for rapid enzymatic quantification of sugars in potato tubers. Potato Research 35:55-58
Visser R.G.F. (1991). Refeneration and transformation of potato by Agrobacterium tumefaciens. In: (ed K. Lindsey) Plant Tissue Culture Manual, B5 pp 1-9, Kluwer Academic Press
Weiner M, Stitt M and Heldt HW (1987) Subcellular compartmentation of pyrophosphate and alkaline pyrophosphatase in spinach leaves. Biochim Biophys Acta 893:13-21

## Claims

1. A method for reducing plant material's ability for producing sugars which can participate in Maillard reactions or precursors of said sugars, wherein at least one nucleotide sequence is introduced into the plant material, said nucleotide sequence expressing a product capable of reducing the amount of UDPG available for conversion to sucrose and wherein the reduction in sugar production occurs upon application of an external stimulus.

2. A method according to claim 1, wherein a temperature below the threshold value for cold sweetening characteristic for the plant material is applied and maintained as external stimulus.

3. A method according to claim 1 or 2, wherein the external stimulus is a temperature below 8°C, preferably below 6°C.

4. A method according to any of the preceding claims, wherein the nucleotide sequence expresses a product capable of inhibiting conversion of UDPG to sucrose by increasing the amount of available cytosolic pyrophosphate.

5. A method according to any of the preceding claims, wherein the nucleotide sequence expresses a product capable of inhibiting conversion of UDPG to sucrose by inhibiting conversion of fructose-6-phosphate to fructose-1,6-P2 via pyrophosphate fructosephosphate phosphotransferase (PFP).

6. A method according to any of the preceding claims wherein at least one nucleotide sequence encoding at least a part of anti-sense pyrophosphate fructosephosphate phosphotransferase (PFP) mRNA is introduced into the plant material, said part being sufficient to cause inhibition of production of pyrophosphate fructosephosphate phosphotransferase (PFP).

7. A method according to any of the preceding claims, wherein the nucleotide sequence is controlled by a promoter that is inducible by the external stimulus that is applied.

8. A method according to any of the preceding claims, wherein the nucleotide sequence is controlled by a tuber specific promoter.

9. Recombinant plant material exhibiting reduced sucrose formation characterised by the presence of at least one nucleotide sequence encoding at least a part of anti-sense pyrophosphate fructosephosphate phosphotransferase (PFP) mRNA, said part being sufficient to cause inhibition of production of pyrophosphate fructosephosphate phosphotransferase (PFP) after application of an external stimulus.

10. Recombinant plant material according to claim 9, wherein said plant material is plant material of a potentially economically interesting agricultural crop.

11. Recombinant plant material according to claim 9 or 10, wherein said plant material is potato material.

12. Recombinant plant material according to any of claims 9-11, wherein the nucleotide sequence is controlled by a tuber specific promoter.

13. Use of a nucleotide sequence encoding at least a part of anti-sense pyrophosphate fructosephosphate phosphotransferase (PFP) mRNA, for obtaining recombinant plant material exhibiting reduced sucrose formation, said part being sufficient to ensure inhibition of production of pyrophosphate fructosephosphate phosphotransferase (PFP).
